# EUROPÄISCHE PATENTANMELDUNG

(11) **EP 2 657 224 A1**
(43) Veröffentlichungstag der Anmeldung: **30.10.2013**
(21) Anmeldenummer: 12165874.4
(22) Anmeldetag: 27.04.2012
(51) Int. Cl.: C07C 253/30, C07C 255/07

(54) **Verfahren zur Isomerisierung von cis-2-Pentennitril zu 3-Pentennitril**

(71) Anmelder: BASF SE, 67056 Ludwigshafen (DE)
(72) Erfinder: Die Erfindernennung liegt noch nicht vor
(74) Vertreter: Baier, Martin

(57) **Zusammenfassung**

Die vorliegende Erfindung betrifft ein verbessertes Verfahren zur diskontinuierlichen oder kontinuierlichen Isomerisierung von cis-2-Pentennitril zu 3-Pentennitrilen in Gegenwart von tertiären Aminen der Formel I als Katalysatoren.

## Beschreibung

Die vorliegende Erfindung betrifft ein verbessertes Verfahren zur diskontinuierlichen oder kontinuierlichen Isomerisierung von cis-2-Pentennitril zu 3-Pentennitrilen in Gegenwart von tertiären Aminen der Formel I als Katalysatoren.

Aus der WO-A-05/73176 ist bekannt, cis-2-Pentennitril mit Hilfe von homogen gelösten Aminen als Katalysatoren, ausgewählt aus der Gruppe C₁- bis C₂₀-Mono- und Diamine, zu 3-Pentennitrilen zu isomerisieren.

Aus der US-A-5 070 202 ist bekannt, dass cis-2-Pentennitril bei Temperaturen von 20 bis 200 °C mit primären und sekundären Aminen Michael-Addukte bildet.

Nachteilig an diesen Isomerisierungen ist, dass sich aus cis-2-Pentennitril und den genannten Aminen als Katalysatoren Michael-Addukte bilden.

Aus WO 2011/055353 ist auch schon bekannt, tertiäre Amine als Katalysatoren für die Isomerisierung von cis-2-Pentennitril zu 3-Pentennitrilen zu verwenden. WO 2011/055353 lehrt, dass die Verwendung von Triethylamin als Katalysator zu einem Umsatz von cis-2-Pentennitril zu 3-Pentennitrilen von nur 13 % beträgt.

Es stellte sich die Aufgabe, Katalysatoren zur Verfügung zu stellen, mit deren Hilfe höhere Ausbeuten an 3-Pentennitrilen bei gleichzeitig hohen 3-Pentennitril-Selektivitäten erzielbar sind.

Es wurde ein verbessertes Verfahren zur Isomerisierung von cis-2-Pentennitril zu 3-Pentennitrilen entwickelt, welches dadurch gekennzeichnet ist, dass cis-2-Pentennitril mit tertiären Aminen der Formel I oder ihren Gemischen isomerisiert wird.

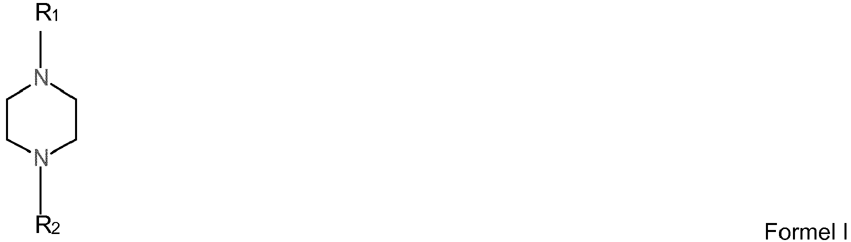

In Formel I können R₁ und R₂ gleich oder verschieden sein. Als R₁ und/oder R₂ können Wasserstoff, lineare und/oder verzweigte Alkylreste mit von einem bis fünf Kohlenstoffatomen, Cycloalkylreste mit von fünf bis sieben Kohlenstoffatomen und/oder Hydroxyethylreste gewählt werden. Bevorzugt ein tertiäres Amin der Formel I bei dem nur einer der Reste R₁ und R₂ ein Wasserstoffatom darstellt. In einer Ausführungsform sind die Reste R₁ und R₂ eine Ethylengruppe, die die beiden Stickstoffatome überbrückt.

Das erfindungsgemäße Verfahren kann wie folgt durchgeführt werden:
Cis-2-Pentennitril kann mit einem tertiären Amin der Formel I als Katalysator bei Temperaturen von 80 bis 200 °C, bevorzugt 90 bis 150 °C, besonders bevorzugt 100 bis 150 °C isomerisiert werden.

Es kann ein Druck von 0,01 bis 50 bar, bevorzugt von 0,1 bis 30 bar, besonders bevorzugt von 0,5 bis 20 bar, insbesondere Normaldruck (Atmosphärendruck) gewählt werden.

Die Isomerisierung kann in diskontinuierlicher oder kontinuierlicher Fahrweise durchgeführt werden. Bevorzugt wird die Isomerisierung kontinuierlich durchgeführt.

Bei der Isomerisierung von cis-2-Pentennitril (Siedepunkt 127 °C/1013 mbar) entstehen die Zielprodukte trans-3-Pentennitril (Siedepunkt 143 °C/1013 mbar) und cis-3-Pentennitril (Siedepunkt 146 °C/1013 mbar), außerdem in geringen Mengen 4-Pentennitril (Siedepunkt 146 °C/1013 mbar) und trans-2-Pentennitril (Siedepunkt 144 °C/1013 mbar). Trans-3-Pentennitril, cis-3-Pentennitril und 4-Pentennitril können nach destillativer Abtrennung für die Hydrocyanierung mit Blausäure zu Adipodinitril eingesetzt werden. Cis- und trans-2-Pentennitril lassen sich nach destillativer Abtrennung in die Isomerisierungsstufe zurückführen.

Die tertiären Amine der Formel I werden bevorzugt so gewählt, dass ihr Siedepunkt höher liegt als der Siedepunkt von cis-3-Pentennitril. Insbesondere werden die tertiären Aminen der Formel Iso gewählt, dass ihr Siedepunkt von 1 bis 100 °C, bevorzugt von 2 bis 50 °C, besonders bevorzugt von 3 bis 30 °C höher liegt als der Siedepunkt von cis-3-Pentennitril.

Das Molverhältnis der tertiären Amine der Formel I zu cis-2-Pentennitril kann in weiten Grenzen variiert werden und beträgt in der Regel von 0,1:1 bis 1:1 Mol, bevorzugt von 0,1:1 bis 0,5:1, besonders bevorzugt von 0,15:1 bis 0,3:1 Mol.

Der Reaktionsaustrag der Isomerisierung kann destillativ aufgearbeitet werden. Nicht umgesetztes cis-2-Pentennitril kann als Kopfprodukt abgetrennt und rückgeführt werden. Trans-3-und cis-3-Pentennitril können nach Abtrennung und Rückführung von höher als cis-3-Pentennitril siedenden Katalysatoren zur Pentennitril-Hydrocyanierung verwendet werden.

Als cis-2-Pentennitril eignen sich reines cis-2-Pentennitril, Mischungen, die cis-2-Pentennitril enthalten, oder Nebenproduktströme der Hydrocyanierung von 1,3-Butadien, die cis-2-Pentennitril, bevorzugt mehr als 50 Gew.-% cis-2-Pentennitril, besonders bevorzugt mehr als 70 Gew.-% cis-2-Pentennitril enthalten.

Als Katalysatoren für die Isomerisierung eignen sich tertiäre Amine der Formel I oder deren Gemische. Die Katalysatoren werden im Folgenden auch als Stickstoffbasen bezeichnet.

Als tertiäre Amine der Formel I sind beispielsweise N-Methylpiperazin, N-Ethylpiperazin, n-Propylpiperazin, N-i-Propylpiperazin, N-n-Butylpiperazin, N-i-Butyl-piperazin, N-sek.-Butylpiperazin, N-tert.-Butylpiperazin, N.N-Dimethylpiperazin, N.N-Diethylpiperazin, N-Methyl-N-ethylpiperazin, N.N-Di-n-propylpiperazin, N.N.-Di-i-propylpiperazin, N.N-Di-n-butylpiperazin, N.N.-Di-i-butylpiperazin, N.N.-Di-sek.-butylpiperazin, N.N.-Di-tert.-butylpiperazin, N-Ethyl-N-cyclohexypiperazin, N-Hydroxyethyl-piperazin, 1.4-Diazabicyclo[2.2.2]octan (DABCO) oder Gemische dieser Amine der Formel I geeignet.

Besonders bevorzugt ist 1.4-Diazabicyclo[2.2.2]octan (DABCO) (Siedepunkt 174 °C/1013 mbar), das sich z. B. durch Erhitzen von N-Hydroxyethylpiperazin herstellen lässt.

Cis-2-Pentennitril, das Ausgangsprodukt für die Isomerisierung zu 3-Pentennitrilen, entsteht beispielsweise bei der Hydrocyanierung von 3-Pentennitrilen zu Adipodinitril.

Die zweistufige Herstellung von Adipodinitril, ausgehend von Butadien und Blausäure ist bekannt (Hans-Jürgen Arpe, Industrielle Organische Chemie, 6. Auflage 2007, Wiley-VCH Verlag, Seiten 272 bis 273).

Im ersten Reaktionsschritt wird Butadien in der Flüssigphase in Gegenwart von Nickel(0)-tritolylphosphit-Komplexverbindungen als Katalysatoren hydrocyaniert. Man isoliert ein Gemisch isomerer Pentennitrile und Methylbutennitrile, insbesondere 3-Pentennitrile und 2-Methyl-3-butennitril. 2-Methyl-3-butennitril wird zu 3-Pentennitrilen isomerisiert.

Im zweiten Reaktionsschritt wird 3-Pentennitril in der Flüssigphase mit Blausäure hydrocyaniert. Man arbeitet in Gegenwart der gleichen Nickel(0)-tritolylphosphit-Komplexe, denen eine LewisSäure wie Zinkchlorid hinzugefügt wird.

Aus dem Hydrocyanierungs-Austrag werden Adipodinitril, weitere Dinitrile und der Nickel(0)-Katalysator-Komplex abgetrennt. Der Katalysator wird, gegebenenfalls nach Regenerierung, in die Hydrocyanierung zurückgeführt.

Die bei der Aufarbeitung erhaltene organische Phase enthält im Wesentlichen ungesättigte C₅-Mononitrile, ausgewählt aus der Gruppe cis-2-Pentennitril, trans-3-Pentennitril, cis-3-Pentennitril, 4-Pentennitril, trans-2-Pentennitril, cis-2-Methyl-2-butennitril, trans-2-Methyl-2-butennitril, 2-Methyl-3-butennitril.

Dieser C₅-Mononitrilstrom kann 0,1 bis 10 Gew.-% cis-2-Pentennitril, insbesondere 1 bis 5 Gew.-% cis-2-Pentennitril enthalten.

Für die diskontinuierliche Isomerisierung kann ein derart gewonnenes cis-2-Pentennitril zunächst destillativ aus dem C₅-Mononitril-Gemisch abgetrennt werden. Für die Isomerisierung kann ein cis-2-Pentennitril mit einer Reinheit von größer 50 Gew.-%, bevorzugt größer 70 Gew.-%, eingesetzt werden, wobei sich die Gewichtsprozente auf die Summe aller Komponenten des Gemisches beziehen.

In einer besonders bevorzugten Ausführungsform kann die Isomerisierung kontinuierlich in integrierter Fahrweise mit im Vergleich zu cis-2-Pentennitril höher siedenden tertiären Aminen der Formel I als Katalysatoren durchgeführt werden. Unter integrierter Fahrweise ist dabei zu verstehen, dass cis-2-Pentennitril, Gemische aus 3-Pentennitril und trans-2-Pentennitril und das tertiäre Amin der Formel I kontinuierlich in die jeweiligen Verfahrensstufen zurückgeführt werden, das heißt, dass alle Rückführungs-Ströme geschlossen sind.

Ein kontinuierliches, integriertes Verfahren zur Isomerisierung von cis-2-Pentennitril zu 3-Pentennitrilen kann folgende Schritte umfassen:
a) 3-Pentennitrile oder eine Mischung enthaltend 3-Pentennitrile werden in Gegenwart von Nickel(0)-Phosphorligand-Komplexen als Katalysatoren zu Adipodinitril hydrocyaniert.
b) Aus dem Hydrocyanierungsaustrag wird Adipodinitril, 2-Methylglutarnitril und Nickel(0)-Phosphorligand-Komplex abtrennt,
c) Das cis-2-Pentennitril wird in der so erhaltenen, im wesentlichen ungesättigte C5-Mononitrile wie cis- und trans-3-Pentennitril, 4-Pentennitril, trans-2-Pentennitril, cis-2-Methyl-2-butennitril, trans-2-Methyl-2-butennitril, 2-Methyl-3-butennitril, enthaltenen organischen Phase mit Hilfe eines tertiären Amins der Formel I als Katalysator, das bei Normaldruck höher als cis-2-Pentennitril siedet, zu 3-Pentennitrilen isomerisiert,
   dadurch gekennzeichnet, dass
d) Die in c) erhaltene, im Wesentlichen ungesättigte C5-Mononitrile enthaltende organische Phase, wird einer Destillationskolonne K1 zugeführt. Die Destillationskolonne K1 kann von 20 bis 40 theoretische Böden besitzen. Die Destillationskolonne K1 kann bei einer Sumpftemperatur im Bereich von 70 bis 145°C betrieben werden. Die Destillationskolonne K1 kann bei einem Druck von 100 bis 1000 mbar betrieben werden. Aus der Destillationskolonne K1 wird über Kopf cis-2-Pentennitril, cis- und trans-2-Methyl-2-butennitril abgetrennt. Über den Sumpf kann 3-Pentennitril und trans-2-Pentennitril abtrennt werden und in den Reaktionsschritt der Hydrocyanisierung von 3-Pentennitrilen (Schritt a)) zurückführt werden.

In einer Ausführungsform kann aus einem Seitenabzug 3-Pentennitrile und trans-2-Pentennitril abgezogen werden und in Schritt a) zurückgeführt werden. In einer Ausführungsform können Hochsieder als Sumpfprodukte ausgeschleust werden.
e) Das Kopfprodukt der Kolonne K1 wird einer Destillationskolonne K2 zugeführt. Die Kolonne K2 kann von 20 bis 40 theoretische Böden besitzen. Die Kolonne K2 kann von bei einer Sumpftemperatur im Bereich von 60 bis 140°C betrieben werden. Die Kolonne K2 kann von bei einem Druck von 50 bis 100 mbar betrieben werden. Das Kopfprodukt der Kolonne K2 enthält cis-2-Pentennitril und cis-2-Methyl-2-butennitril. Als Sumpfprodukt der Kolonne K2 wird trans-2-Methyl-2-butennitril abgetrennt und ausgeschleust. Außerdem wird der Kolonne K2 so viel tertiäres Amin I zugeführt, dass Amin-Verluste im Kreislauf, die durch Ausschleusung zustande kommen, ausgeglichen werden.
f) Das Kopfprodukt der Kolonne K2 und ein höher als cis-2-Pentennitril siedendes tertiäres Amin der Formel I wird einem Reaktor R1 zuführt. Das Reaktionsprodukt des Reaktors R1 und das höher als cis-2-Pentennitril siedende tertiäre Amin der Formel I wird einer Destillationskolonne K3 zuführt.
g) Der Reaktionsaustrag des Reaktors R1, der 3-Pentennitrile als Isomerisierungsprodukte, trans-2-Pentennitril, cis-2-Methyl-2-butennitril, nicht umgesetztes cis-2-Pentennitril und tertiäres Amin I enthält, wird einer Kolonne K3 zugeführt. Die Kolonne K3 kann von 10 bis 20 theoretische Böden umfassen. Die Kolonne K3 kann bei einer Sumpftemperatur im Bereich von 100 bis 150°C betrieben werden. Die Kolonne K3 kann bei einem Druck von 100 bis 1000 mbar, betrieben werden.

Das Kopfprodukt der Kolonne K3 wird in den Reaktor R1 zurückgeführt. Ein Teilstrom des Kopfproduktes wird ausgeschleust, um eine Aufpegelung von cis-2-Methyl-2-butennitril zu verhindern. Aus einem Seitenabzug wird ein Strom entnommen, der neben überwiegend cis-2-Pentennitril, trans-3-Pentennitril, trans-2-Methyl-2-butennitril enthält. Dieser Strom wird auf die Kolonne K1 zurückgeführt. Als Sumpfprodukt werden tertiäres Amin der Formel I und gegebenenfalls Hochsieder ausgeschleust. Das tertiäre Amin der Formel I kann gegebenenfalls nach Abtrennung der Hochsieder, z. B. in einer Kolonne oder einem Dünnschichtverdampfer, in den Reaktor zurückgeführt werden.

In einer bevorzugten Ausführungsform der Erfindung wird der Reaktor R1 durch eine Reaktionskolonne ersetzt. In WO-A-05/73177 sind Reaktionskolonnen für die Isomerisierung von cis-2-Pentennitril zu 3-Pentennitrilen in Gegenwart von homogenen und heterogenen Katalysatoren beschrieben.

Die kontinuierliche Isomerisierung von cis-2-Pentennitril zu 3-Pentennitrilen unter Verwendung von höher als 2-cis-Pentennitril siedenden tertiären Aminen der Formel I kann in, dem Fachmann bekannten, Vorrichtung durchgeführt werden. Für die Destillation geeignet sind Vorrichtungen, wie sie beispielsweise in: Kirk-Othmer, Encyclopedia of Chemical Technology, 4. Ed., Vol. 8, John Wiley & Sons, New York, 1996, Seiten 334 bis 338, beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet. Die Destillation kann dabei in mehreren, wie zwei oder drei Vorrichtungen, durchgeführt werden. Die Destillation kann zudem einstufig im Sinne einer Teilverdampfung des Zulaufstroms erfolgen. Für die Isomerisierung kommen übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055, beschrieben sind, wie Rührkesselreaktoren, Schlaufenreaktoren, Gasumlaufreaktoren, Blasensäulenreaktoren oder Rohrreaktoren, bevorzugt Rohrreaktoren, jeweils gegebenenfalls mit Vorrichtungen zur Wärmeübertragung. Die Reaktion kann in mehreren, wie zwei oder drei, Apparaturen durchgeführt werden.

Besonders bevorzugt für die Isomerisierung ist eine Rührkesselkaskade mit 2 bis 4, insbesondere 3 Rührkesseln oder ein Strömungsrohr.

Der Isomerisierungsreaktor wird bei Temperaturen von 100 bis 150°C, bevorzugt 100 bis 130°C und Drucken von 1 bis 20 bar betrieben. Die Verweilzeiten im Reaktor betragen 0.5 bis 5 Stunden, bevorzugt 1 bis 3 Stunden. Der cis-2-Pentennitril-Umsatz beträgt dabei 5 bis 30, bevorzugt 10 bis 25, besonders bevorzugt 10 bis 20 %.

Das erfindungsgemäße Verfahren für die Isomerisierung wird vorzugsweise in einer Destillationskolonne, mindestens umfassend eine Sumpfzone, eine Reaktionszone und eine Kopfzone, durchgeführt. Dabei sind Sumpfzone, Reaktionszone und Kopfzone in der vorgegebenen Reihenfolge von unten nach oben in der Destillationskolonne angeordnet. Es ist dabei nicht ausgeschlossen, dass auch in der Sumpf- oder Kopfzone noch Reaktion stattfindet.

Zusätzlich kann die Destillationskolonne Einbauten mit destillativer Trennwirkung umfassen. Diese zusätzlichen Einbauten sind vorzugsweise unter- und/oder oberhalb der Reaktionszone angeordnet. In der unteren Trennzone, d.h. der Trennzone unterhalb der Reaktionszone, wird das schwer siedende Isomerisierungsprodukt weitgehend von leicht siedenden Komponenten abgetrennt. So werden z. B. trans-2-Pentennitril und trans-3-Pentennitril von nicht umgesetztem cis-2-Pentennitril getrennt. In der oberen Trennzone, d.h. der Trennzone oberhalb der Reaktionszone, werden leicht siedende Nebenkomponenten weitgehend von schwer siedenden Komponenten abgetrennt. So wird hier beispielsweise gegebenenfalls mit dem Eduktstrom eingetragenes trans-2-Methyl-2-butennitril von trans-3-Pentennitril und trans-2-Pentennitril getrennt. Ebenso kann aus nicht isomerisiertem cis-2-Pentennitril trans-3-Pentennitril und trans-2-Pentennitril abgereichert werden. Diese Trennungen sind nur beispielhaft aufgeführt und sind nicht einschränkend.

Bei optimaler Kolonnenkonfiguration kann so das gesamte cis-2-Pentennitril des Eduktstroms ohne zusätzlichen Reaktor umgesetzt werden und das gesamte trans-3-Pentennitril im Sumpf ohne zusätzlichen Trennapparat erhalten werden. Dabei sind die zusätzlichen Einbauten mit destillativer Trennwirkung (Trennzonen) im Allgemeinen von Vorteil, aber nicht zwingend notwendig. So kann auch eine der beiden oder beide Trennzonen entfallen.

Die Reaktionszone besteht im Allgemeinen aus mehreren unterschiedlichen Teilbereichen mit unterschiedlichen Funktionen. Die Teilbereiche unterscheiden sich durch die Aufgabe des Transportes von Gas zum Kopf der Kolonne und die Aufgabe des Ableitens von Flüssigkeit in Richtung des Kolonnensumpfes. Zudem können Flüssigkeitsverteiler innerhalb der Reaktionszone notwendig sein, um eine optimale Verteilung von Flüssigkeit über den Kolonnenquerschnitt zu gewährleisten. Auch Einbauten zum Eintragen von Wärme in die Kolonne können sich in der Reaktionszone befinden.

### Beispiele

### Beispiel 1

### Isomerisierung von cis-2-Pentennitril mit 1.4-Diazabicyclo[2.2.2]octan (DABCO) als Katalysator

Das als Einsatzstoff verwendete C₅-Mononitril-Gemisch wurde nach WO-A-05/73172, Beispiel 1, durch Hydrocyanierung von 3-Pentennitril in Gegenwart von Ni(0)Komplexen, die ausgehend von einer Ligandmischung aus 60 mol-% Tri(m/p-tolyl)phosphit und 40 mol-% des Chelatphosphoniten 1 synthetisiert worden waren, und Zinkchlorid hergestellt. Nach Abtrennung von Adipodinitril, weiterer Dinitrile und des Ni(0)-Katalysators wurde ein C₅-Nitril-Gemisch erhalten, das neben trans-3-Pentennitril (trans-3PN), cis-3-Pentennitril (cis-3PN) und 4-Pentennitril (4PN) weiterhin cis-2-Methyl-2-butennitril (cis-2M2BN) (5 %), trans-2-Methyl-2-butennitril (trans-2M2BN) (2 %), cis-2-Pentennitril (cis-2PN) (5 %) und trans-2-Pentennitril (trans-2PN) enthielt.

Die Prozentangaben beziehen sich auf die Summe aller genannten ungesättigten Nitrile.

### Chelatphosphonit 1

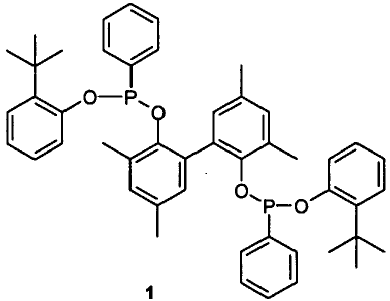

Das C₅-Gemisch wurde einer Kolonne K1 zugeführt, die 30 theoretische Trennstufen besaß und bei einer Sumpftemperatur von 79°C und einem Druck von 100 mbar betrieben wurde. Über Kopf gingen cis-2-Pentennitril und cis- und trans-2-Methyl-2-butennitril. Als Sumpfprodukte wurden 3-Pentennitril und trans-2-Pentennitril abgezogen und in den Schritt a), die Hydrocyanierung von 3-Pentennitril, zurückgeführt.

Das Kopfprodukt der Kolonne K1, das 79 Gew.-% cis-2-Pentennitril und 10 Gew.-% 2-Methyl-2-butennitrile enthielt, wurde der Kolonne 2 zugeführt, die 33 theoretische Trennstufen besaß und bei einer Sumpftemperatur von 72°C und einem Druck von 100 mbar betrieben wurde.

Das Kopfprodukt der Kolonne K2 enthielt 86 Gew.-% cis-2-Pentennitril und 3 Gew.-% trans-2-Methyl-2-butennitril. Als Sumpfprodukt der Kolonne K2 wurde ein Strom ausgeschleust, der 61 Gew.-% trans-2-Methyl-2-butennitril enthielt.

Das Kopfprodukt der Kolonne K2 wurde einem Reaktorsystem R-1 zugeführt, das aus einer Kaskade von drei Rührkesseln bestand. In R-1 fand der überwiegende Teil der cis-2-Pentennitril-Isomerisierung bei einer Verweilzeit von 4 Stunden statt. Katalysator-Verluste, z. B. durch Ausschleusung, konnten über eine Zuleitung von 1.4-Diazabicyclo[2.2.2]octan (DABCO) ausgeglichen werden. Die Isomerisierungstemperatur betrug 125°C, der Druck 2 bar. Das Molverhältnis von cis-2-Pentennitril zu 1.4-Diazabicyclo[2.2.2]octan (DABCO) lag bei 1 zu 0,5.

Der Reaktionsaustrag des Reaktorsystems R-1 wurde einer Kolonne K3 zugeführt, die 15 theoretische Trennstufen besaß und bei einer Sumpftemperatur von 110°C und einem Druck von 250 mbar betrieben wurde.

Das Kopfprodukt der Kolonne K3 wurde in das Reaktorsystem zurückgeführt. Ein Teilstrom des Kopfprodukts (ca. 5 Gew.-%), das cis-2-Methyl-2-buten (cis-2M2BN) und cis-2-Pentennitril enthält, wurde ausgeschleust. Ein Seitenabzugsprodukt, das zu 79 Gew.-% aus cis-2-Pentennitril bestand, wurde auf die Kolonne K1 zurückgeführt. Über Sumpf wurden tert. Amin Ia und gegebenenfalls Hochsieder ausgeschleust. Das tert. Amin Ia konnte nach Abtrennung der Hochsieder, in den Reaktor R1 zurückgeführt werden.

### Beispiel 2

Isomerisierung von cis-2-Pentennitril (cis-2PN) zu cis- und trans-3-Pentennitril (cis-+trans-3PN) in Gegenwart von 1.4-Diazabicyclo[2.2.2]octan (DABCO) als Katalysator

Ein mit Stickstoff inertisierter 100 ml Kolben wurde mit 16,2 g (0,2 mol) cis-2-PN (99,6 %ig) befüllt, auf 120 °C aufgeheizt und unter Rühren mit DABCO (0,04 mol) versetzt. Das Reaktionsgemisch wurde 6 Stunden lang bei 119 bis 121 °C gerührt. Nach 2, 4 und 6 Stunden wurden Proben gezogen und gaschromatographisch analysiert (GC-Säule: 30 m ZB 50, 25 µl, erst 11 Minuten isotherm bei 40 °C, dann Temperaturprogramm bis 280 °C, 10 °C pro Minute).

Tabelle 1 zeigt GC-Flächen-%-Werte für cis-+trans-2-PN und cis-+trans-3-PN + 4-Pentennitril (4-PN), jeweils bezogen auf die GC-Flächensumme aller fünf linearen Pentennitril-Isomeren.

| tert. Amin | Reaktionszeit [h] | GC-Flächen-% | |
|---|---|---|---|
| | | cis-+trans 2-PN | cis-+trans 3-PN + 4-PN |
| | 2 | 55,0 | 45,0 |
| DABCO | 4 | 44,9 | 55,1 |
| | 6 | 39,4 | 60,6 |

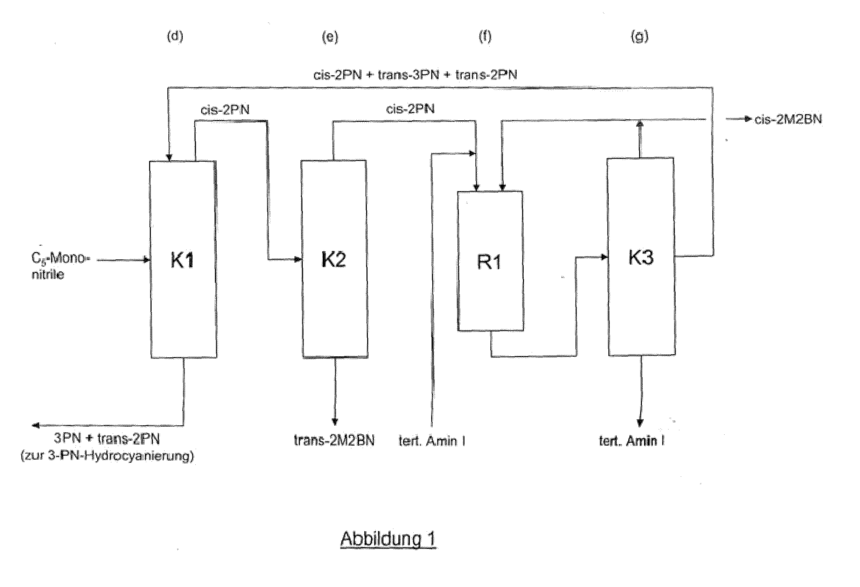

## Patentansprüche

1. Verfahren zur diskontinuierlichen oder kontinuierlichen Isomerisierung von cis-2-Pentennitril zu 3-Pentennitrilen, **dadurch gekennzeichnet, dass** man cis-2-Pentennitril mit tertiären Aminen der Formel I oder ihren Gemischen isomerisiert, 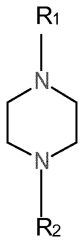 wobei R₁ und R₂ gleich oder verschieden sind und Wasserstoff, lineare oder verzweigte Alkylreste mit von einem bis fünf Kohlenstoffatomen, Cycloalkylreste mit von fünf bis sieben Kohlenstoffatomen oder Hydroxyethylreste bedeuten können oder R₁ und R₂ eine Ethylengruppe sind, die die beiden Stickstoffatome überbrückt.

2. Verfahren nach Anspruch 1 wobei cis-2-Pentennitril bei Temperaturen von 80 bis 200 °C und einem Druck von 0,01 bis 50 bar isomerisiert wird.

3. Verfahren nach Anspruch 1 oder 2 wobei ein Katalysator der Formel I mit einem Siedepunkt eingesetzt wird, der von 1 bis 100 °C höher ist als der Siedepunkt von cis-3-Pentennitril.

4. Verfahren nach mindestens einem der Ansprüche 1 bis 3 wobei als Katalysator 1.4-Diazabicyclo[2.2.2]octan einsetzt wird.

5. Verfahren nach mindestens einem der Ansprüche 1 bis 4 wobei die Isomerisierung von cis-2-Pentennitril zu 3-Pentennitrilen in einer Reaktionskolonne durchgeführt wird.

6. Verfahren nach mindestens einem der Ansprüche 1 bis 5, wobei
a) 3-Pentennitrile oder eine Mischung enthaltend 3-Pentennitrile in Gegenwart von Nickel(0)-Phosphorligand-Komplexen als Katalysatoren zu Adipodinitril hydrocyaniert werden,
b) aus dem Hydrocyanierungsaustrag Adipodinitril, 2-Methylglutarnitril und Nickel(0)-Phosphorligand-Komplex abtrennt wird,
c) das cis-2-Pentennitril in der so erhaltenen, im Wesentlichen umgesättigte C5-Mononitrile wie cis- und trans-3-Pentennitrile, 4-Pentennitril, trans-2-Pentennitril, cis-2-Methyl-2-butennitril, trans-2-Methyl-2-butennitril, 2-Methyl-3-butennitril, enthaltenden organischen Phase mit Hilfe eines tertiären Amins der Formel I als Katalysator, die bei Normaldruck höher als cis-2-Pentennitril siedet, zu 3-Pentennitrilen isomerisiert wird,
**dadurch gekennzeichnet, dass**
d) die in c) erhaltene, im Wesentlichen ungesättigte C5-Mononitrile enthaltende organische Phase einer Destillationskolonne K1 zugeführt wird, aus der Destillationskolonne K1 über Kopf cis-2-Pentennitril, cis- und trans-2-Methyl-2-butennitril, über Sumpf 3-Pentennitril und trans-2-Pentennitril abgetrennt wird und in den Reaktionsschritt der Hydrocyanierung von 3-Pentennitrilen zurückgeführt wird,
e) das Kopfprodukt der Kolonne K1 einer Destillationskolonne K2 zugeführt wird, als Sumpfprodukt der Kolonne K2 trans-2-Methyl-2-butennitril abgetrennt und ausgeschleust wird,
f) das Kopfprodukt der Kolonne K2 und ein bei Normaldruck höher als cis-2-Pentennitril siedendes tertiäres Amin der Formel I einem Reaktor R1 zugeführt wird, das Reaktionsprodukt des Reaktors R1 und das höher als cis-2-Pentennitril siedende tertiäre Amin der Formel I einer Destilationskolonne K3 zugeführt wird,
g) das Kopfprodukt der Destillationskolonne K3 in den Reaktor R1 zurückgeführt wird und ein Teilstrom des Kopfprodukts, der cis-2-Methyl-2-butennitril und cis-2-Pentennitril enthält, ausgeschleust wird, aus einem Seitenabzug ein Gemisch aus cis-2-Pentennitril und trans-3-Pentennitril, trans-2-Pentennitril in Destillationskolonne K1 zurückgeführt und als Sumpfprodukt tertiäres Amin der Formel I und gegebenenfalls Hochsieder ausgeschleust werden.

7. Verfahren zur kontinuierlichen Isomerisierung von cis-2-Pentennitril zu 3-Pentennitrilen nach Anspruch 6, **dadurch gekennzeichnet, dass** man die Isomerisierung von cis-2-Pentennitrilen in einer Reaktionskolonne durchführt.

8. Verwendung von tertiären Aminen der Formel I oder deren Gemische als Katalysatoren für die Isomerisierung von cis-2-Pentennitril zu 3-Pentennitril.
